# FASCICULE DE BREVET EUROPEEN

(11) **EP 1 263 489 B1**
(45) Date de publication et mention de la délivrance du brevet: **05.04.2006**
(21) Numéro de dépôt: 01903971.8
(22) Date de dépôt: 26.01.2001
(51) Int. Cl.: A61M 5/30

(54) **SERINGUE SANS AIGUILLE POUR L'INJECTION D'UN LIQUIDE CONTENU DANS UNE AMPOULE PRE-REMPLIE**
NADELLOSE SPRITZE ZUR VERABREICHUNG VON IN EINER VORGEFÜLLTEN AMPULLE BEINHALTETEN FLÜSSIGKEIT
NEEDLELESS SYRINGE FOR INJECTING A LIQUID CONTAINED IN A PREFILLED AMPULE

(30) Priorité: 11.02.2000 FR 0001721
(43) Date de publication de la demande: 11.12.2002
(73) Titulaire: Crossject, 75181 Paris Cedex 04 (FR)
(72) Inventeur: ALEXANDRE, Patrick, F-70100 Gray (FR); BROUQUIERES, Bernard, F-83100 Toulon (FR); CASTANO, Xavière, F-21000 Dijon (FR); GAUTIER, Philippe, F-91220 Le Plessis Pate (FR)
(74) Mandataire: Maureau, Philippe
(86) Numéro de dépôt international: PCT/FR2001/000250
(87) Numéro de publication internationale: WO 2001/058512

(56) Documents cités:
- EP-A- 0 888 791
- WO-A-00/48654
- FR-A- 2 775 603
- US-A- 4 668 223
- US-A- 6 004 287

## Description

La présente invention est dans le domaine des seringues sans aiguille, pré-remplies et jetables ; de telles seringues sont utilisées pour les injections intradermiques, sous-cutanées et intramusculaires, de principe actif liquide à usage thérapeutique en médecine humaine ou vétérinaire.

Un premier impératif pour des seringues pré-remplies est celui de la compatibilité à long terme, trois ans en général, entre le principe actif liquide et le réservoir qui le contient. Un autre impératif, lié au procédé de pré-remplissage, est d'avoir un réservoir transparent pour faire les contrôles réglementaires du remplissage correct du réservoir avant son montage dans la seringue. Ces impératifs conduisent à la réalisation de réservoir essentiellement transparent et en matériau compatible avec le principe actif pour la durée souhaitée : c'est en général du verre à usage pharmaceutique : verre de type I ou II.

La phase initiale de l'injection est critique pour la pénétration dans la peau du jet ou des jets de liquide, suivant que la seringue a un ou plusieurs conduits d'injection. Cette dernière configuration étant favorable pour réduire la douleur. La biodisponibilité finale dépend de la bonne réalisation de cette phase initiale, elle suppose une mise en vitesse rapide du liquide dans les conduits d'injection sans les saccades multiples des jets quand il y a un coup de bélier trop important pour réaliser cette mise en vitesse rapide.

L'état de la technique ne présente pas de seringues, à plusieurs conduits d'injection, répondant à tous ces besoins.

Le brevet US 4 941 880 décrit une seringue fort complexe de plusieurs points de vue. D'abord il s'agit d'une seringue à moteur pneumatique, devant être utilisée plusieurs fois par changement de l'ampoule contenant le principe actif. Cette ampoule contenant le principe actif comporte deux parties juxtaposées. Une partie amont, pour le stockage de longue durée du principe actif : c'est un tube de verre fermé à ses deux extrémités par des obturateurs et placé dans un tube de polycarbonate : cette disposition assure la compatibilité et permet les contrôles de remplissage. Avant l'utilisation, l'opérateur transfère le principe actif dans la partie aval de l'ampoule qui joue le rôle de réservoir intermédiaire, en repoussant un piston qui déplace le liquide et l'obturateur aval ; cette partie aval du réservoir est en polycarbonate et à paroi épaisse, elle va résister à la pression de fonctionnement générée par le dispositif moteur. L'opérateur place l'ampoule, ainsi préparée pour l'injection, dans le mécanisme d'injection ; en amont d'un dispositif d'injection comportant un seul conduit d'injection qui sera alimenté par un by-pass placé à l'extrémité aval du réservoir intermédiaire d'injection. Cette disposition résout les problèmes de résistance du réservoir et, dans une certaine mesure peut réduire les saccades initiales de l'injection, mais avec des pertes de charges importantes dans un système d'injection tortueux pour alimenter un seul conduit d'injection. La complexité du dispositif tant dans sa réalisation que dans son utilisation est évidente.

Le brevet FR 2 775 603 décrit une seringue sans aiguille avec une ampoule pré-remplie fermée à son extrémité amont par un obturateur déplaçable et à son extrémité aval par un injecteur multitrou comportant un orifice central de remplissage fermé par un bouchon après remplissage de l'ampoule.

Le brevet US 4 668 223 décrit une seringue avec une ampoule pré-remplie fermée à chacune de ses extrémités par un obturateur déplaçable, l'extrémité amont de l'ampoule reçoit une pièce de guidage d'une tige de piston et l'extrémité aval une pièce formant réceptacle dans laquelle vient se loger l'obturateur aval.

La présente invention vise à résoudre tous ces problèmes par un dispositif le plus simple possible, jetable après l'injection et utilisable non seulement par du personnel spécialisé mais aussi par un patient ordinaire.

La présente invention concerne une seringue sans aiguille comportant un réservoir essentiellement cylindrique fermé par un obturateur amont déplaçable, un obturateur aval déplaçable et emprisonnant un principe actif liquide, le réservoir étant initialement isolé d'un système d'injection solidaire d'un corps, ladite seringue comportant un moyen moteur pour déplacer l'ensemble : obturateur amont - liquide - obturateur aval est caractérisée en ce que le système d'injection comporte au moins deux conduits périphériques d'injection qui sont situés à l'extérieur d'un réceptacle de l'obturateur aval, la hauteur libre de l'alésage borgne du réceptacle permettant le dégagement des entrées des conduits périphériques lorsque cet obturateur aval est au contact du fond du réceptacle.

Dans cette invention par principe actif liquide, ou médicament, nous entendrons essentiellement un liquide plus ou moins visqueux, ou un mélange de liquides, ou un gel. Le principe actif pourra être un solide mis en solution dans un solvant approprié pour l'injection. Le principe actif pourra être un solide sous forme pulvérulente mis en suspension, plus ou moins concentrée, dans un liquide approprié. La granulométrie du principe actif solide doit être adaptée, ainsi que la forme du conduit pour éviter les bouchages.

Le réservoir, essentiellement cylindrique, est en verre de type I ou de type II ; mais il peut être en tout autre matériau transparent et compatible avec le principe actif.

Le moyen moteur qui va agir sur l'obturateur amont peut être un moteur mécanique : détente d'un ressort comprimé ou du type pneumatique : détente de gaz comprimé, ou pyrotechnique : détente de gaz de combustion.

Le fonctionnement de la seringue est le suivant : le moyen moteur va agir sur l'obturateur amont et déplacer l'ensemble obturateur amont - liquide - obturateur aval puisque le liquide est incompressible. L'obturateur aval se déplace et se loge dans l'alésage borgne du réceptacle jusqu'au contact avec le fond dudit réceptacle. La hauteur de cet alésage est telle que lorsque l'obturateur aval est au contact du fond du réceptacle les entrées des conduits d'injection, sur la périphérie du réceptacle, sont dégagées ; le liquide y est refoulé et est injecté par le mouvement de l'obturateur amont qui se poursuit jusqu'à la vidange du réservoir : l'obturateur amont est alors au contact de l'obturateur aval.

La seringue sans aiguille est telle que le réservoir de principe actif liquide est logé dans le corps sur lequel est fixé le système d'injection.

Le réceptacle, dans lequel se loge l'obturateur aval, est une partie du corps de la seringue.

Le réceptacle de l'obturateur aval comporte au moins un moyen d'amortissement de l'impact de l'obturateur aval avant son arrêt dans le fond du réceptacle. Cet amortissement, qui évite les rebonds de l'obturateur a pour but d'éviter l'injection par saccades au début du fonctionnement.

Selon une première variante l'obturateur aval comporte, sur sa face tournée vers le réceptacle, un évidement de forme conjuguée à celle d'un plot solidaire du fond du réceptacle. Pendant son déplacement l'obturateur aval va s'emmancher par cet évidement sur le plot. Cet emmanchement absorbe l'énergie d'impact de l'obturateur sur le fond du réceptacle. De plus cet emmanchement est rendu irréversible, par exemple, par un bourrelet ou des crans disposés autour du plot.

Selon une autre variante, l'obturateur aval peut venir se loger dans un réceptacle de forme très légèrement tronconique avec une grande section à l'entrée du réceptacle et une section plus petite au fond. La déformation de l'obturateur pour se loger dans ce réceptacle amortit l'impact.

Selon une autre variante l'obturateur aval comporte une partie compressible. L'obturateur aval vient se loger dans l'alésage du réceptacle en écrasant cette partie compressible : la profondeur de l'alésage est inférieure à la hauteur de l'obturateur avant sa déformation. C'est l'écrasement de cette partie déformable qui amortit l'impact de l'obturateur aval.

Selon une première réalisation le moyen moteur agit directement sur l'obturateur amont.

Selon une autre réalisation le moyen moteur agit sur l'obturateur amont par l'intermédiaire d'un piston. Cette variante est intéressante lorsque.le moyen moteur est un générateur du type pneumatique ou du type pyrotechnique. En utilisant alors un piston étagé, à la place d'un piston simple, on réalise une première phase à faible pression et faible vitesse pour déplacer l'ensemble « obturateur amont - liquide - obturateur aval » et engager, sans choc l'obturateur aval dans le réceptacle puis, une deuxième phase à forte pression pour l'injection à grande vitesse.

Préférentiellement le moyen moteur est un générateur pyrotechnique de gaz. Un tel dispositif est compact, puissant et surtout très fiable : la durée de stockage de ce type de générateur est largement supérieure à la période de conservation du principe actif.

Avantageusement le réservoir et le corps de la seringue forment un ensemble unique, compatible pour une longue durée avec le principe actif et résistant à des pressions élevées lors du fonctionnement.

Selon une autre réalisation le réservoir est fretté dans le corps par un matériau intermédiaire lors du montage, sur le moyen moteur, du corps de la seringue avec le réservoir.

Avantageusement le corps de la seringue est réalisé dans un matériau transparent ce qui permet la visualisation du réservoir de principe actif jusqu'au moment de l'injection.

Toutefois le corps comporte au moins une fenêtre de visualisation du contenu du réservoir, lorsque le matériau du corps de la seringue n'est pas transparent.

Avantageusement le piston agissant sur l'obturateur amont sert d'indicateur du fonctionnement de la seringue en apparaissant dans la partie transparente ou la fenêtre du corps de la seringue. L'obturateur amont peut aussi remplir cette fonction.

La seringue sans aiguille avec plusieurs conduits d'injection dans les différentes réalisations de l'invention résout les problèmes posés. La compatibilité à long terme entre le principe actif liquide et le réservoir le contenant par le choix de la forme et des matériaux du réservoir. La tenue du réservoir à la forte pression de fonctionnement par les différents type de montage du réservoir dans le corps. La réalisation d'une injection sans saccades au début de la phase de mise en vitesse du liquide par le fonctionnement du dispositif et l'amortissement de l'impact.

L'air, en très faible quantité, initialement emprisonné dans les conduits d'injection n'introduit aucun effet néfaste. De même le principe actif qui reste dans les conduits en fin d'injection se traduit par une perte très négligeable de principe actif qui peut même être compensée au moment du pré-remplissage.

La présente invention a l'avantage de permettre de séparer, dans le dispositif, deux parties. Une partie qui sera dite partie pharmaceutique comprenant le corps et le réservoir avec les obturateurs déplaçables amont et aval : ce sous-ensemble pourra être traité dans les conditions de l'industrie pharmaceutique notamment en ce qui concerne la stérilisation et l'asepsie. Ce sous-ensemble sera intégré au reste de la seringue, dont les éléments ont été assemblées par ailleurs, cet assemblage se faisant dans les conditions moins sévères que celles liées à l'industrie pharmaceutique.

Lorsque l'obturateur aval est logé de façon irréversible dans le réceptacle la seringue devient très difficilement réutilisable. Cette disposition a donc aussi l'avantage d'empêcher des réutilisations de ladite seringue à des fins différentes de l'utilisation thérapeutique initiale.

Enfin cette configuration présente l'avantage d'éviter des fuites éventuelles de liquide par les conduits d'injection avant la réalisation de l'injection. En effet l'agitation du dispositif est fréquemment réalisée, voire préconisée pour examiner la turbidité du liquide ou homogénéiser le mélange lorsque le liquide comporte des particules en suspension. Le fait que le principe actif soit isolé, avant injection, des conduits réalise une protection ultime vis à vis de ce risque de perte.

Ci-dessus l'invention est exposée en détail à l'aide de figures représentant différentes réalisations particulières de l'invention.

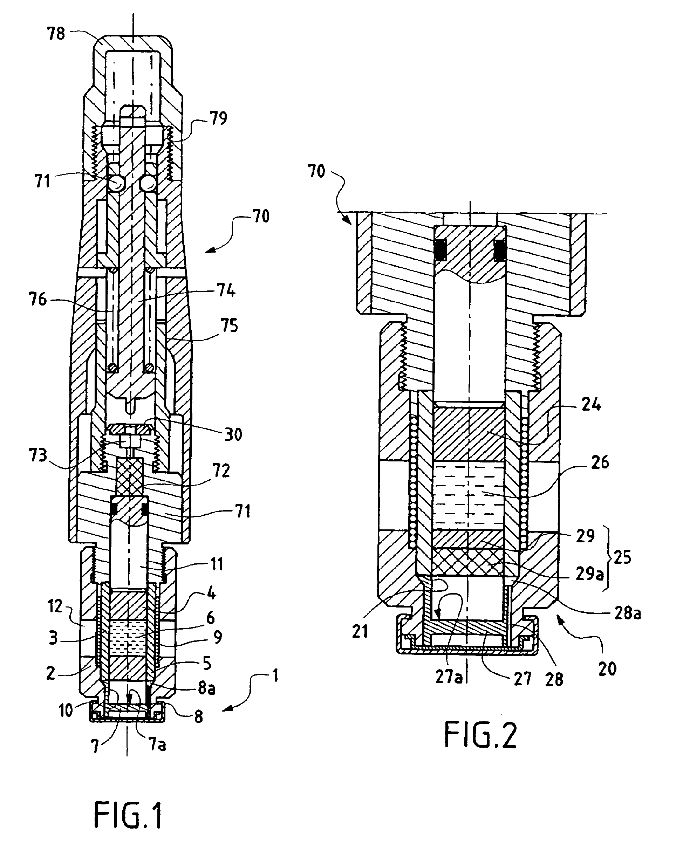

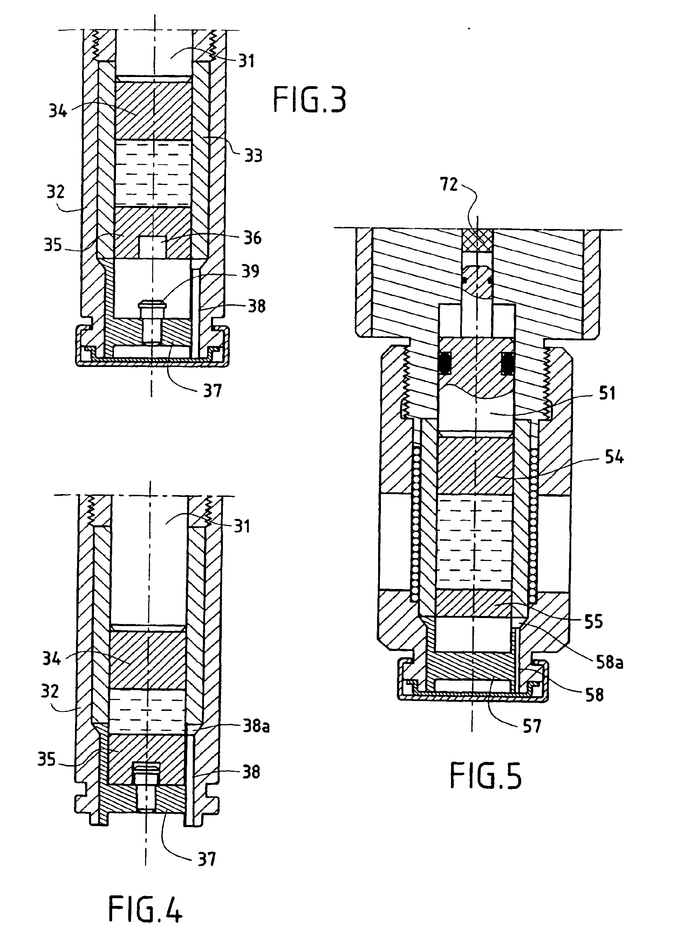

La figure 1 représente une coupe longitudinale d'une seringue selon une première réalisation. La figure 2 présente la vue partielle de l'extrémité aval d'une seringue dans laquelle l'obturateur aval a une partie compressible. Les figures 3 et 4 représentent une vue partielle de l'extrémité aval d'une seringue selon une autre variante, respectivement avant et après fonctionnement, dans cette variante l'obturateur aval comporte un évidement. La figure 5 représente une variante avec un piston étagée pour déplacer les obturateurs amont et aval.

La figure 1 représente en coupe longitudinale partielle une seringue selon l'invention, elle est représentée verticale, le système d'injection dirigé vers le bas.

La seringue 1 comporte un corps 2 dans lequel est logé un réservoir 3 contenant le principe actif liquide 6. A l'extrémité aval du corps 2 est placé un réceptacle 7 comportant, par exemple, trois conduits d'injection tels que le conduit 8. Le système d'injection est recouvert d'une protection extérieure pour assurer l'asepsie de la seringue : cette protection comprend une membrane d'élastomère appliquée sur la face extérieure de l'injecteur par un opercule métallique fin, serti autour de cette extrémité de la seringue. Cette protection sera retirée avant l'injection. A son extrémité opposée, le corps 2 de la seringue est fixé à un moyen moteur 70 qui, dans cet exemple, est un générateur pyrotechnique de gaz, il sera décrit par la suite.

Le corps 2 de la seringue comporte deux fenêtres diamétralement opposées pour la visualisation du principe actif contenu dans le réservoir 3 : ce sont simplement deux ouvertures oblongues 12 dans le corps. A l'aval du corps 2 de la seringue est emmanché, dans un alésage de forme appropriée, un réceptacle 7 cylindro-conique qui sera décrit par la suite. En appui sur ce réceptacle 7 et centré dans l'aval du corps 2 est positionné un réservoir 3 de verre ; ce réservoir est un tube entouré d'un matériau intermédiaire 9 transparent. En amont le corps 2 de la seringue reçoit le corps 71 du moyen moteur qui se centre autour de l'autre extrémité du réservoir, la couronne annulaire de centrage vient appuyer sur le matériau intermédiaire 9 et frette ainsi le réservoir 3 dans le corps 2 au moment du montage du moyen moteur sur le corps 2 de la seringue. Ce frettage augmente la résistance du tube lorsqu'il est soumis à la pression de fonctionnement. Le réservoir 3 est essentiellement un tube fermé à ses deux extrémités par des obturateurs déplaçables amont 4 et aval 5 ; ces obturateurs sont préférentiellement des bouchons-pistons habituellement utilisés dans les seringues : ce sont des pièces obtenues par moulage d'élastomères compatibles pour une longue durée avec le principe actif : chaque pièce intègre les fonctions de piston et d'étanchéité par la réalisation de bourrelets ou de lèvres (non détaillée sur les figures). Les élastomères habituellement utilisés pour la fabrication de ces pièces sont par exemple des chlorobutyl ou bromobutyl, dont la dureté Shore est réglée entre environ 45 et environ 70. Ces pièces peuvent recevoir des traitements de surface notamment pour faciliter leurs déplacements dans le réservoir tubulaire. Lorsqu'il est libre, le bouchon-piston a un diamètre supérieur d'environ 10 pour cent au diamètre intérieur du tube qui va le recevoir, la hauteur du bouchon-piston est d'environ 0,5 à 0,8 fois ce diamètre. Lorsque le bouchon-piston est engagé dans le tube, du fait des déformations, sa hauteur est égale à environ 0,6 fois à environ 1,0 fois le diamètre intérieur du réservoir.

Le réceptacle 7 est dans cet exemple une pièce de forme extérieure cylindro-conique qui comporte un alésage central 10 dans lequel va venir se loger l'obturateur aval 5. Sur sa périphérie le réceptacle comporte trois conduits d'injection dont un seul, repère 8, est visible sur cette coupe. Le diamètre de l'alésage est égal à celui du réservoir. La hauteur libre de l'alésage borgne 10 du réceptacle 7 est égale à celle de l'obturateur aval 5 monté dans le réservoir 3. Lorsque l'obturateur aval 5 a atteint le fond 7a du réceptacle, l'entrée 8a (côté réservoir 3) des conduits d'injection 8 est mise en communication avec le liquide 6 ; le liquide s'écoule avec une vitesse correspondant à la pression transmise par l'obturateur amont 4.

Dans cette réalisation le moyen moteur agit sur l'obturateur amont par l'intermédiaire d'un piston 11 de section efficace égale à celle de l'obturateur amont 5. Ce piston 11 est en contact avec l'obturateur amont 5 il n'y a donc pas d'effet de choc ou de bélier en début de fonctionnement. Ce piston 11 grâce à son système d'étanchéité empêche les gaz produits par la combustion du chargement 72 de venir en contact avec l'obturateur amont et donc d'éventuelles détériorations de celui-ci et des fuites de gaz vers le principe actif contenu dans le réservoir. Ce piston 11, d'une couleur adaptée, peut aussi servir d'indicateur de fonctionnement en apparaissant dans les fenêtres de visualisation du corps 2 de la seringue.

Nous allons décrire les principaux éléments, du générateur pyrotechniques 70. Il comprend dans le corps 71 au dessus du piston un chargement pyrotechnique 72 dont la combustion est initiée par une amorce 80 impactée par un percuteur 74. L'amorce 73 est logée dans un porte-amorce. En position initiale le percuteur 74 est retenu, dans le guide-percuteur 75 solidaire par vissage du corps 71, par au moins une bille, telle que la bille 77, partiellement engagée dans une gorge du percuteur. Le dispositif de percussion comprend un poussoir 78 avec une gorge 79 et un ressort intérieur 76.

Le poussoir 78 coulisse sur l'extérieur du guide-percuteur 75 et il est retenu pas des ergots se déplaçant dans des rainures latérales. Ce poussoir 78 est ici l'organe de déclenchement.

Bien entendu pour initier la combustion du chargement pyrotechnique 72, sans sortir du cadre de l'invention, on peut utiliser des dispositifs d'initiation autre que le dispositif à percuteur ici décrit. Sans entrer dans les détails et sans vouloir être exhaustif, nous citerons comme exemples des dispositifs d'initiation à pile électrique ou des dispositifs d'initiation piézo-électrique.

Eventuellement le générateur de gaz pyrotechnique peut être remplacé par un générateur de gaz constitué par un réservoir de gaz comprimé fermé par une vanne à ouverture rapide. L'organe de déclenchement va ouvrir ladite vanne, les gaz comprimés du réservoir vont se détendre et agir sur le moyen de poussée.

Pour l'utilisation, après avoir enlevé le bouchon d'asepsie, et posé la face aval de l'injecteur sur la peau du sujet à traiter, l'opérateur appuie, avec son pouce, sur le poussoir 78 qui s'enfonce en comprimant le ressort 76. Le poussoir se déplace jusqu'à ce que la gorge 78 arrive à la hauteur de la gorge du percuteur 74, les billes, telle que la bille 77, retenant le percuteur 74, se dégagent dans la gorge 79 et libèrent le percuteur qui va impacter violemment l'amorce 73, dont l'initiation enflamme le chargement pyrotechnique 72. Le percuteur 74 en appui sur le porte-amorce 80 assure le maintien en place de l'amorce et l'étanchéité : les gaz de combustion ne remontant pas vers le poussoir.

La combustion du chargement pyrotechnique va produire des gaz qui agissent sur le piston 11.

La figure 2 présente la vue partielle d'une autre réalisation de l'invention dans laquelle l'obturateur aval 25 est partiellement compressible. Cet obturateur avant fonctionnement a une hauteur ou épaisseur supérieure à la hauteur de l'alésage 21 borgne du réceptacle 27. Comme précédemment le moyen moteur déplace l'ensemble obturateur amont 24 - liquide 26 - obturateur aval 25 ; l'obturateur aval se loge dans l'alésage borgne 21 du réceptacle puis vient au contact du fond et sous l'effet de la pression, la partie compressible s'écrase libère les entrées des conduits d'injection et permet le passage du liquide. La déformation par compression partielle de l'obturateur aval 25 dans l'alésage borgne du réceptacle amortit le choc dû à l'arrêt de l'obturateur aval et évite l'éjection du liquide par saccades multiples. L'obturateur aval 25 est constitué, par exemple, de deux couches de matériaux différents : côté réservoir une couche d'élastomère déformable 29 mais incompressible, compatible avec le principe actif, puis dessous une couche d'un matériau très compressible 29a qui va s'écraser lorsque l'obturateur aval s'engage jusqu'au fond du réceptacle.

Dans une variante l'obturateur aval est réalisé dans un même élastomère compatible avec le principe actif mais comporte sur sa partie inférieure, venant en contact avec le fond du réceptacle, au moins une cavité qui sera fermée par la déformation de l'élastomère lors de l'engagement de l'obturateur aval dans le réceptacle. Cette déformation amortit l'impact de l'obturateur et réduit aussi le volume occupé par l'obturateur.

La figure 3 représente, en vue partielle, une autre réalisation de l'invention qui diffère des précédentes par la forme particulière de l'obturateur aval et par le montage du réservoir dans le corps de la seringue.

Le corps 32 de la seringue est réalisé dans un matériau transparent et suffisamment épais pour résister a de fortes pressions de fonctionnement. Un réceptacle 37 cylindro-conique est emmanché dans l'extrémité aval du corps 32. Le réservoir 33, contenant le principe actif liquide et fermé par les obturateurs amont 34 et aval 35, est logé dans le corps 32. Le corps 32 sert de renfort au réservoir 33 au moment de l'injection.

Une autre particularité de cet exemple est l'évidement 36 sur la face aval de l'obturateur déplaçable 35 et le plot 39 fixé dans le fond du réceptacle. L'évidemment 36 et le plot 39 ont des formes qui permettent leur emboîtement lorsque l'obturateur aval 35 se déplace sous l'effet de la pression transmise par le piston 31. Cet emboîtement absorbe de l'énergie et amortit l'impact de l'obturateur 35 sur le fond du réceptacle 37 : il n'y a pas de rebond de l'obturateur aval 35 qui reste emmanché sur le plot 39.

L'amortissement de l'obturateur aval dans le réceptacle peut aussi être réalisé par un réceptacle de forme légèrement tronconique dans lequel vient se loger et se déformer l'obturateur aval.

L'amortissement peut aussi être réalisé pneumatiquement par au moins un trou évent calibré qui contrôle le débit d'air refoulé par l'obturateur aval lors de son déplacement dans le réceptacle.

La figure 4 représente, en vue partielle, la position des obturateurs aval 35 et amont 34 pendant l'injection. L'obturateur aval 35 est dans l'alésage borgne du réceptacle 37, il est engagé par son évidement 36 sur le plot 39 fixé sur le fond du réceptacle : cet emmanchement est rendu irréversible par un bourrelet entourant le plot 39. L'obturateur amont 34, poussé par le piston 31, refoule le liquide. Cette figure 4 illustre bien une injection en cours : l'obturateur 35 est en appui sur le fond du réceptacle 37 et il libère les entrées 38a des conduits d'injection 38 et cela, tant que l'obturateur amont 34 n'a pas terminé lui aussi sa course pour venir en appui sur l'obturateur aval 35.

La figure 5 présente une autre réalisation de l'invention qui diffère de l'exemple de la figure 2 par l'utilisation d'un piston étagé 51. Côté générateur de gaz, le piston 51 comporte une tête de petite section qui va transmettre une force modérée, en début de fonctionnement, pour la mise en mouvement et l'accélération de l'ensemble « obturateur amont 54 - liquide 56 - obturateur aval 55 ». Les longueurs de déplacement sont choisies de telle façon que lorsque la tête de petite section se dégage, plus précisément lorsque le joint supérieur du piston 51 cesse d'être efficace, l'obturateur aval 55 est entièrement logé dans le réceptacle 57, les gaz du générateur vont alors agir sur la grande section du piston 51 soumettant le liquide 56 à une forte pression et comme alors il est mis en communication avec les ouvertures 58a des conduits d'injection 58 le liquide 56 va être injecté à grande vitesse ce qui est favorable au percement de la peau et à une bonne biosdisponibilité du principe actif.

## Revendications

1. Seringue sans aiguille (1) comportant un corps (2) logeant un réservoir cylindrique (3,33), fermé par un obturateur amont déplaçable (4,24,34,54) et un obturateur aval déplaçable (5,25,35,55) emprisonnant un principe actif liquide (6) et comportant à l'aval un réceptacle (7,27,37,57) avec au moins deux conduits périphériques d'injection (8) qui sont placés à l'extérieur dudit réceptacle, ledit réceptacle comprenant un alésage borgne (10,20,21) dont la hauteur libre permet le dégagement des entrées (8a) des conduits périphériques (8) lorsque l'obturateur aval (2,25,35,55) est amené au contact du fond (7a) dudit réceptacle par le fonctionnement d'un moyen moteur (70), faisant partie de la seringue et déplaçant l'ensemble obturateur amont-liquide-obturateur aval, ladite seringue est **caractérisée en ce que** le réceptacle comporte un moyen d'amortissement de l'obturateur aval (5,35) avant son arrêt dans le réceptacle (7,37).

2. Seringue sans aiguille selon la revendication 1 **caractérisée en ce que** le réceptacle (37) comporte un plot (39) sur lequel va s'emmancher, par un évidement (36), l'obturateur aval (35).

3. Seringue sans aiguille selon la revendication 1 **caractérisée en ce que** l'obturateur aval (25) est déformable.

4. Seringue sans aiguille selon la revendication 3 **caractérisée en ce que** le réceptacle (27) est plus petit que l'obturateur aval (25) avant sa déformation.

5. Seringue sans aiguille selon l'une des revendications 1 à 4 **caractérisée en ce que** le moyen moteur (70) agit directement sur l'obturateur amont (4,24,34,54).

6. Seringue sans aiguille selon l'une des revendications 1 à 4 **caractérisée en ce que** le moyen moteur (70) agit sur l'obturateur amont (4,24,34,54) par l'intermédiaire d'un piston (11,31,51).

7. Seringue sans aiguille selon l'une des revendications précédentes **caractérisée en ce que** moyen moteur (70) est un générateur pyrotechnique de gaz.

8. Seringue sans aiguille selon l'une des revendications 1 à 7 **caractérisée en ce que** le réservoir et le corps forment un ensemble unique.

9. Seringue sans aiguille selon l'une des revendications 1 à 7 **caractérisée en ce que** le réservoir (3) est fretté dans le corps (2) par un matériau intermédiaire (9) lors du montage sur le moyen moteur (70).

10. Seringue sans aiguille selon l'une des revendications 1 à 9 **caractérisée en ce que** le corps (32) est transparent.

11. Seringue sans aiguille selon l'une des revendications 1 à 10 **caractérisée en ce que** le corps (2) comporte au moins une fenêtre (12) de visualisation du contenu du réservoir.

12. Seringue sans aiguille selon la revendication 6 **caractérisée en ce que** le piston (51) est un piston étagé pour réaliser une première phase à faible pression et faible vitesse pour déplacer l'ensemble obturateur amont (54) - liquide (56) - obturateur aval (55) et engager sans choc l'obturateur aval dans le réceptacle (57), puis une deuxième phase à forte pression pour l'injection à grande vitesse.

13. Seringue sans aiguille selon la revendication 12 **caractérisée en ce que**, du côté générateur de gaz, le piston étagé (51) comporte une tête de petite section pour produire une force modérée pour la mise en mouvement de l'ensemble obturateur amont (54) - liquide (56) - obturateur aval (55), la longueur de déplacement est telle que lorsque la tête de petite section se dégage, l'obturateur aval (55) est entièrement logé dans le réceptacle (57) et les gaz du générateur vont alors agir sur la grande section du piston (51) : le liquide est soumis à une forte pression.

## Patentansprüche

1. Nadellose Spritze (1) mit einem Körper (2), der ein zylindrisches Reservoir (3, 33) aufnimmt, das von einer verschiebbaren vorderen Verschlussvorrichtung (4, 24, 34, 54) und von einer verschiebbaren hinteren Verschlussvorrichtung (5, 25, 35, 55) verschlossen wird und einen flüssigen Wirkstoff (6) einschließt, und das hinten einen Behälter (7, 27, 37, 57) mit mindestens zwei Injektions-Umfangsleitungen (8) aufweist, die außerhalb des Behälters angeordnet sind, wobei der Behälter eine Blindbohrung (10, 20, 21) aufweist, deren freie Höhe die Freigabe der Eingänge (8a) der Umfangsleitungen (8) ermöglicht, wenn die hintere Verschlussvorrichtung (2, 25, 35, 55) durch den Betrieb einer Antriebseinrichtung (70) mit dem Boden (7a) des Behälters in Kontakt gebracht wird, die Teil der Spritze ist und die Einheit aus vorderer Verschlussvorrichtung - Flüssigkeit - hinterer Verschlussvorrichtung verschiebt, wobei die Spritze **dadurch gekennzeichnet ist, dass** der Behälter eine Dämpfungseinrichtung für die hintere Verschlussvorrichtung (5, 35) vor ihrem Anhalten im Behälter (7, 37) aufweist.

2. Nadellose Spritze nach Anspruch 1, **dadurch gekennzeichnet, dass** der Behälter (37) einen Stopfen (39) aufweist, auf den sich die hintere Verschlussvorrichtung (35) durch eine Aussparung (36) hindurch aufschiebt.

3. Nadellose Spritze nach Anspruch 1, **dadurch gekennzeichnet, dass** die hintere Verschlussvorrichtung (25) verformbar ist.

4. Nadellose Spritze nach Anspruch 3, **dadurch gekennzeichnet, dass** der Behälter (27) kleiner ist als die hintere Verschlussvorrichtung (25) vor ihrer Verformung.

5. Nadellose Spritze nach einem der Ansprüche 1 bis 4, **dadurch gekennzeichnet, dass** die Antriebseinrichtung (70) direkt auf die vordere Verschlussvorrichtung (4, 24, 34, 54) einwirkt.

6. Nadellose Spritze nach einem der Ansprüche 1 bis 4, **dadurch gekennzeichnet, dass** die Antriebseinrichtung (70) mittels eines Kolbens (11, 31, 51) auf die vordere Verschlussvorrichtung (4, 24, 34, 54) einwirkt.

7. Nadellose Spritze nach einem der vorhergehenden Ansprüche, **dadurch gekennzeichnet, dass** die Antriebseinrichtung (70) ein pyrotechnischer Gasgenerator ist.

8. Nadellose Spritze nach einem der Ansprüche 1 bis 7, **dadurch gekennzeichnet, dass** das Reservoir und der Körper ein einziges Bauteil bilden.

9. Nadellose Spritze nach einem der Ansprüche 1 bis 7, **dadurch gekennzeichnet, dass** der Behälter (3) bei der Montage auf die Antriebseinrichtung (70) über ein Zwischenmaterial (9) in den Körper (2) geschrumpft wird.

10. Nadellose Spritze nach einem der Ansprüche 1 bis 9, **dadurch gekennzeichnet, dass** der Körper (32) durchsichtig ist.

11. Nadellose Spritze nach einem der Ansprüche 1 bis 10, **dadurch gekennzeichnet, dass** der Körper (2) mindestens ein Sichtfenster (12) für den Inhalt des Reservoirs aufweist.

12. Nadellose Spritze nach Anspruch 6, **dadurch gekennzeichnet, dass** der Kolben (51) ein gestufter Kolben ist, um eine erste Phase mit geringem Druck und geringer Geschwindigkeit, um die Einheit aus vorderer Verschlussvorrichtung (54), Flüssigkeit (56) und hinterer Verschlussvorrichtung (55) zu verschieben und die hintere Verschlusseinrichtung ohne Aufprall in den Behälter (57) einzuführen, und dann eine zweite Phase mit starkem Druck für die Injektion mit großer Geschwindigkeit auszuführen.

13. Nadellose Spritze nach Anspruch 12, **dadurch gekennzeichnet, dass** der gestufte Kolben (51) auf der Seite des Gasgenerators einen Kopf mit kleinem Querschnitt aufweist, um eine gemäßigte Kraft für das Inbewegungsetzen der Einheit aus vorderer Verschlussvorrichtung (54) - Flüssigkeit (56) - hinterer Verschlussvorrichtung (55) zu erzeugen, wobei die Länge der Verschiebung derart ist, dass, wenn der Kopf mit kleinem Querschnitt sich löst, die vordere Verschlussvorrichtung (55) vollständig im Behälter (57) angeordnet ist und die Gase des Generators dann auf den großen Querschnitt des Kolbens (51) einwirken: die Flüssigkeit wird einem starken Druck unterworfen.

## Claims

1. A needleless syringe (1) including a body (2) housing a cylindrical reservoir (3,33) which is closed by a displaceable upstream obturator (4,24,34,54) and by a displaceable downstream obturator (5,25,35,55) confining a liquid active ingredient (6), and including upstream a receptacle (7,27,37,57) with at least two peripheral injection ducts (8) which are placed outside said receptacle, said receptacle comprising a blind bore (10,20,21), the free height of which permits the disengagement of the inlets (8a) of the peripheral ducts (8) when the downstream obturator (2,25,35,55) is brought into contact with the bottom (7a) of said receptacle by the operation of a driving means (70) forming part of the syringe and displacing the unit constituted by upstream obturator, liquid and downstream obturator, said syringe being **characterised in that** the receptacle includes a means for cushioning the downstream obturator (5,35) prior to the arrest thereof in the receptacle (7,37).

2. Needleless syringe according to Claim 1, **characterised in that** the receptacle (37) includes a stud (39) on which the downstream obturator (35) will be fixed by means of a recess (36).

3. Needleless syringe according to Claim 1, **characterised in that** the downstream obturator (25) is deformable.

4. Needleless syringe according to Claim 3, **characterised in that** the receptacle (27) is smaller than the downstream obturator (25) prior to deformation thereof.

5. Needleless syringe according to one of Claims 1 to 4, **characterised in that** the driving means (70) acts directly on the upstream obturator (4,24,34,54).

6. Needleless syringe according to one of Claims 1 to 4, **characterised in that** the driving means (70) acts on the upstream obturator (4,24,34,54) via a plunger (11,31,51).

7. Needleless syringe according to one of the preceding claims, **characterised in that** the driving means (70) is a pyrotechnic gas generator.

8. Needleless syringe according to one of Claims 1 to 7, **characterised in that** the reservoir and the body form a single assembly.

9. Needleless syringe according to one of Claims 1 to 7, **characterised in that** the reservoir (3) is hooped within the body (2) by means of an intermediate material (9) at the time of mounting on the driving means (70).

10. Needleless syringe according to one of Claims 1 to 9, **characterised in that** the body (32) is transparent.

11. Needleless syringe according to one of Claims 1 to 10, **characterised in that** the body (2) includes at least one window (12) for viewing the contents of the reservoir.

12. Needleless syringe according to Claim 6, **characterised in that** the plunger (51) is a stepped plunger in order to effect a first phase with low pressure and low speed in order to displace the unit constituted by upstream obturator (54), liquid (56) and downstream obturator (55) and to engage the downstream obturator in the receptacle (57) without impact, and then a second phase with high pressure for injection at high speed.

13. Needleless syringe according to Claim 12, **characterised in that** on the gas-generator side the stepped plunger (51) includes a head of small cross-section in order to produce a moderate force for setting the unit constituted by upstream obturator (54), liquid (56) and downstream obturator (55) in motion, the displacement length being such that when the head of small cross-section is disengaged the downstream obturator (55) is housed entirely within the receptacle (57) and the gases from the generator will then act on the large cross-section of the plunger (51) so that the liquid is subjected to a high pressure.
